(19) Europäisches Patentamt
European Patent Office
Office européen des brevets

(11) **EP 4 386 379 B1**

(12) **EUROPEAN PATENT SPECIFICATION**

(45) Date of publication and mention
of the grant of the patent:
**11.02.2026 Bulletin 2026/07**

(21) Application number: **22856088.4**

(22) Date of filing: **02.08.2022**

(51) International Patent Classification (IPC):
*G01N 33/18* (2006.01)     *G06T 7/246* (2017.01)
*G06T 7/73* (2017.01)      *G16B 40/00* (2019.01)
*G16B 45/00* (2019.01)     *G16B 50/00* (2019.01)
*G06N 20/00* (2019.01)     *G06N 3/08* (2023.01)

(52) Cooperative Patent Classification (CPC):
**G01N 33/186; G01N 35/10; G06N 3/08;
G06N 20/00;** G01N 2035/1032

(86) International application number:
**PCT/KR2022/011362**

(87) International publication number:
**WO 2023/018089 (16.02.2023 Gazette 2023/07)**

(54) **ONLINE CONTINUOUS BIOTOXICITY MEASURING DEVICE**

ONLINE-VORRICHTUNG ZUR KONTINUIERLICHEN MESSUNG DER BIOTOXIZITÄT

DISPOSITIF DE MESURE DE BIOTOXICITÉ CONTINUE EN LIGNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.08.2021 KR 20210106711**

(43) Date of publication of application:
**19.06.2024 Bulletin 2024/25**

(73) Proprietors:
• **Dongmoonent Co., Ltd
Seoul 08377 (KR)**
• **Centennial Technology Company
Ansan-si, Gyeonggi-do 15588 (KR)**

(72) Inventors:
• **LEE, Kyoung Jin
Seoul 08324 (KR)**
• **KIM, Jong Young
Bucheon-si, Gyeonggi-do 14537 (KR)**
• **KAHNG, Sung Hyun
Ansan-si Gyeonggi-do 15480 (KR)**
• **KHANG, Bum Ju
Siheung-si Gyeonggi-do 14989 (KR)**
• **CHOI, Song Bum
Ansan-si Gyeonggi-do 15594 (KR)**

• **YANG, Mi Suk
Ansan-si Gyeonggi-do 15594 (KR)**
• **SEO, Joon Ho
Ansan-si Gyeonggi-do 15484 (KR)**
• **KIM, Seung Ki
Ansan-si Gyeonggi-do 15502 (KR)**
• **BYEON, Ji Yoon
Ansan-si Gyeonggi-do 15534 (KR)**

(74) Representative: **Beck & Rössig
European Patent Attorneys
Denninger Str. 169
81925 München (DE)**

(56) References cited:
CN-A- 109 574 237      KR-B1- 100 337 943
KR-B1- 101 024 036     KR-B1- 101 280 458
KR-B1- 101 366 786     KR-B1- 101 366 786
KR-B1- 101 399 453     US-A- 4 626 992
US-A- 5 916 801

- TOTHILL I E ET AL: "DEVELOPMENTS IN BIOASSAY METHODS FOR TOXICITY TESTING IN WATER TREATMENT", TRAC TRENDS IN ANALYTICAL CHEMISTRY, ELSEVIER, AMSTERDAM, NL, vol. 15, no. 5, 1 May 1996 (1996-05-01), pages 178 - 187, XP000599457, ISSN: 0165-9936, DOI: 10.1016/0165-9936(96) 80640-6

**Description**

Technical Field

The present invention relates to an online continuous biotoxicity monitoring device, and more particularly, to a device that continuously measures the ecotoxicity of harmful substances present in water using aquatic organisms on site.

Background Art

Although wastewater treatment plants are operated to improve water quality and protect the ecosystem, toxic chemicals cannot be completely removed through physical, chemical, and biological treatment processes. The toxic substances that are not removed through wastewater treatment are discharged to public waters, thereby having harmful effects on the ecosystem and health.

It is known that there are more than 345,000 chemicals used around the world and about 44,000 chemicals are in use in Korea. Even trace amount of some water pollutants may have a negative impact on the public health and the ecosystem. Therefore, toxic chemicals should be strictly and thoroughly managed. In Korea, 1,084 chemicals are designated as toxic substances under the domestic law, and among them, effluent standards for wastewater treatment plants are set for only 48 chemicals. Although compliance monitoring is implemented under the laws that regulate point sources discharging pollutants into waters, only a very small number of toxic substances are subject to compliance monitoring.

However, it is not cost-effective to increase the number of toxic substances managed by setting the effluent standards for the wastewater treatment plants. It is impossible and economically unaffordable to control discharges by applying effluent standards to lots of toxic substances. To solve this problem, environmental management agencies around the world establish ecotoxicological assessment and monitoring systems by measuring the acute and chronic toxicity of effluents to living organisms. For example, in the U.S., whole effluent toxicity (WET) is used to measure the toxic effect on survival, growth, and reproduction of specific aquatic organisms from all pollutants contained in a facility's wastewater effluent. In the WET testing, acute and chronic toxicity are measured using adults, larvae, embryos, and eggs of water fleas, shrimps, sea urchins, and fishes.

In Korea, an ecotoxicity monitoring system using water fleas was introduced to manage toxic substances contained in the wastewater effluent. The ecotoxicity is measured by a toxic unit (TU), and effluent standards for wastewater treatment plants are applied to 1 TU or 2 TU depending on the area where wastewater is discharged. TU is calculated by measuring half maximal effective concentration (EC50), which is determined by exposing water flea larvae for 24 hours to an effluent solution (100%) and solutions diluted to 50%, 25%, 12.5%, 6.25%, and 0%, and estimating the concentration expected to have an adverse effect on 50% of test organisms, such as lethal effect or swimming inhibition.

$$\text{Toxic Unit (TU)} = 100 \,/\, \text{EC50 (\%)}$$

The ecotoxicity of a facility's wastewater effluent is 1 TU, which means that 50% of water flea larvae exposed to the effluent solution for 24 hours are killed or immobile. The ecotoxicity of 2 TU means that 50% of water flea larvae exposed to a solution diluted to 50% for 24 hours are killed or immobile.

Private and public wastewater treatment plants that discharge harmful substances should take samples every month and submit them to a testing laboratory to monitor the ecotoxicity of effluent. However, regular sampling and ecotoxicity testing of effluents are not enough to prevent pollution, because the harmful substances are not only generated in the manufacturing processes but also introduced through spill accidents or intentional disposal.

In Korea, an online water quality monitoring system (hereinafter. referred to as water quality TMS) has been operated at the end-of-pipe of wastewater treatment plants since 2008. Water quality of wastewater effluent is automatically measured in real-time and 24/7/365, all the time, without interruption. When illegal discharge is detected, fines are imposed based on the amount exceeding the effluent standards. As of 2022, the water quality TMS has been installed and operated at 1,027 sites nationwide. The online monitoring parameters are total organic carbon, total nitrogen, total phosphorus, pH, total suspended solid, and discharge flow rate.

The real-time data is transmitted to a central control room through Internet communication networks and is provided to organizations such as the Ministry of Environment after data verification. However, ecotoxicity cannot be monitored automatically in real-time because there is no online equipment that measures TU on site. Since a sample is collected once a month, is transferred to a laboratory, and is then manually tested for toxicity, it takes several days to obtain results; as a result, constant surveillance cannot be performed only with intermittent sampling and testing.

In 1986, large-scaled spill accident occurred in Germany, and pesticides and harmful substances were introduced into the Rhine River. Water intake was prohibited in several countries downstream of the Rhine. Thereafter, biological monitoring early warning systems have been developed using living organisms, such as luminescent bacteria, sulfur-oxidizing microorganisms, water fleas, fishes, vegetable algae, bivalve, etc. They continuously and automatically monitor the physiological or behavioral responses of aquatic organisms exposed under flow-through conditions to surface water or wastewater and provide an immediate indication of abnormal organism responses. Online biotoxicity monitors produce a toxicity index (TI) as an indicator. TI is a relative value that does not have an absolute unit, because it is obtained by simply converting the responses of the organisms induced by the toxic substances into electrical signals or numerical values. TI represents only a relative change in the biological responses over time after the organisms are exposed to the toxic substances. When a spill accident occurs, inflow of the toxic substances may be detected only by monitoring the changes of TI.

However, there is a significant problem that TI cannot be used for administrative purposes for compliance monitoring by setting the effluent standards for the wastewater treatment plant. This is because TI is a relative value that may change depending on the condition of the equipment, health of test organisms, and environmental status. For compliance monitoring, it is necessary to use TU, which is calculated from EC50. Because EC50 is an actual concentration at which test organisms are killed or immobile, after the effluent is sequentially diluted and is exposed to test organisms for a certain period of time.

Meanwhile, KR 102317085 B1 discloses an ecotoxicity monitoring system and method in which movement of water fleas is observed in real time through a video of a camera, a "change" between the movements of the water fleas over time in sample water or a "difference" between the movements of the water fleas in the sample water and a standard water are detected through an image analysis technology and an "arithmetic and statistical operation" of the movement, and thus whether toxic substances area contained in the sample water is monitored.

However, KR 10-2317085 B1 is an invention related to a system that determines only inflow of the toxic substances through cross-exposure of the standard water and the sample water, is regardless of the ecotoxicity value TU and does not disclose a detailed structure of the system.

KR 101366786 B1 discloses an automated toxicity test device using a water flea and an automated toxicity test evaluation method using the same, the automated toxicity test device including a housing, a sample storing unit which is provided inside the housing and in which a sample to be measured is stored, a dilution water storing unit that stores dilution water for diluting the sample, a mixer to which the sample is supplied from the sample storing unit, to which the dilution water is supplied from the dilution water storing unit, and in which the sample and the dilution water are mixed, an automatic dispensing unit that dispenses the sample, of which a dilution ratio of the sample and the dilution water is differently adjusted, from the mixer to each test container, an optical imaging unit that takes a video of activity of the water flea input into the test container, a vibration unit that applies vibration to the test container, a transfer unit that guides movement of the automatic dispensing unit and the optical imaging unit, a controller that sets a mixing ratio of the sample supplied through a moving path of the transfer unit and the automatic dispensing unit, and an analysis unit that evaluates toxicity of the sample through the photographed image transmitted from the optical imaging unit.

However, KR 101366786 B1 has a limitation in that the device is a batch type device that automates manual experiments in a laboratory (measured once 24 hours after 60 water fleas are input), and thus continuous real-time monitoring and warning of the ecotoxicity cannot be performed.

Disclosure

Technical Problem

The present invention is directed to providing a device that may remotely measure ecotoxicity of an effluent from a wastewater treatment plant and a wastewater discharge workplace 24 hours a day.

Technical Solution

One aspect of the present invention provides an online continuous biotoxicity monitoring device including a housing, a supply unit including a sample storing unit in which a sample is stored and a culture medium storing unit in which a culture medium that dilutes the sample is stored, a continuous dilution unit configured to mix the sample and the culture medium supplied from the sample storing unit and the culture medium storing unit at various mixing ratios, including a sample storing tank in which the sample supplied from the sample storing unit is stored, a culture medium storing tank in which the culture medium supplied from the culture medium storing unit is stored, and a plurality of dilution valves connected to a plurality of sample dispensing lines dispensed from the sample storing tank and a plurality of culture medium dispensing lines dispensed from the culture medium storing tank and controlled to supply the sample or the culture medium at different dilution ratios, a chamber part in which an aquatic organism is accommodated and which continuously receives a mixture of the sample and the culture medium through the continuous dilution unit, an optical imaging unit that takes a video of activity of the aquatic organism, and an analysis unit that evaluates toxicity of the sample through the captured image transmitted from the optical imaging unit.

The continuous dilution unit may further include a body extending in a vertical direction, and a plurality of dilution lines passing from a rear side to a front side of the body and connected to the chamber part and the dilution valves, the sample storing tank and the culture medium storing tank may be formed to pass through the body in the vertical direction, the sample dispensing lines may pass from the sample storing tank to a rear side of the body and are spaced apart from each other in the vertical direction, and the culture medium dispensing lines may pass from the culture medium storing tank to the rear side of the body and may be spaced apart from each other in the vertical direction.

The dilution valve may include a first dilution valve to a sixth dilution valve.

The supply unit may further include a unit for storing algae supplied as food for toxicity test organisms.

The supply unit may further include a first supply valve of which an inlet is connected to the sample storing unit and the unit for storing algae and an outlet is connected to the sample storing tank and which is controlled to supply the sample or the algae to the sample storing tank, and a second supply valve of which an inlet is connected to the culture medium storing unit and the unit for storing algae and an outlet is connected to the culture medium storing tank and which is controlled to supply the culture medium or the algae to the culture medium storing tank.

An interior of the chamber part may have a hexagonal shape.

An inlet through which the mixture of the sample and the culture medium is introduced may be provided at a lower end of the chamber part and an outlet through which the mixture of the sample and the culture medium is discharged may be provided at an upper end of the chamber part.

A front side of the chamber part may be made of a transparent material, and a rear side of the chamber part may be made of a translucent material.

The optical imaging unit may include a lighting device disposed behind the chamber part, and an optical camera device that is provided in front of the chamber part and photographs an accommodation space inside the chamber part.

The analysis unit may measure a toxic index (TI) of the sample.

The online continuous biotoxicity monitoring device may further include a prediction unit that calculates a predicted toxic unit (TU) based on the TI measurement value transmitted from the analysis unit.

The prediction unit may calculate the predicted TU by comparing the TI measurement value with previously stored TI data.

Advantageous Effects

According to an embodiment of the present invention, samples having different concentrations can be supplied to a chamber part through a continuous dilution unit having a simple structure, thereby achieving miniaturization of a device.

In addition, a TI of the sample continuously introduced into the chamber part can be measured in real time, a predicted TU at a future target time, that is, after 24 hours, is calculated from a TI measurement value measured at an initial time of introduction of toxic substances, and thus a user can quickly cope with the toxic substances when the toxic substances exceed a discharge standard.

The effects of the present invention are not limited to the above effects and should be understood to include all effects that may be deduced from the detailed description of the present invention or the configuration of the present invention described in the appended claims.

Description of Drawings

FIG. 1 is a perspective view of an online continuous biotoxicity monitoring device according to an embodiment of the present invention.
FIG. 2 is a diagram of the online continuous biotoxicity monitoring device according to the embodiment of the present invention.
FIG. 3 is a perspective view of a continuous dilution unit according to the embodiment of the present invention.
FIG. 4 is a cross-sectional view along direction A-A' of FIG. 3.
FIG. 5 is a cross-sectional view along direction B-B' of FIG. 3.
FIG. 6 is a cross-sectional perspective view along direction C-C' of FIG. 3.

Modes of the Invention

Hereinafter, the present invention will be described with reference to the accompanying drawings. However, the present invention may be implemented in various different forms and thus is not limited to embodiments described herein. Further, in the drawings, parts irrelevant to the description are omitted in order to clearly describe the present invention, and throughout the specification, similar numerals reference numerals are assigned to similar parts.

Throughout the specification, when a first part is connected to a second part, this includes not only a case in which the first part is "directly connected" to the second part but also a case in which the first part is "indirectly connected" to the second part with a third part interposed therebetween. Further, when a part "includes" a component, this means that another component is not excluded but may be further included unless otherwise stated.

The terms including an ordinal number such as "first" or "second" used herein may be used to describe various components or steps, but the components or steps should not be limited by the ordinal number. The terms including an ordinal number should be construed only for distinguishing one component or step from other components or steps.

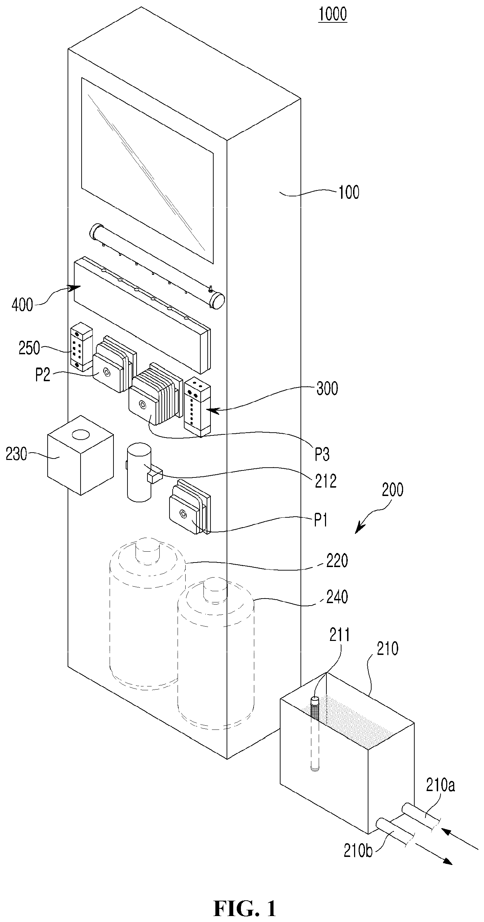

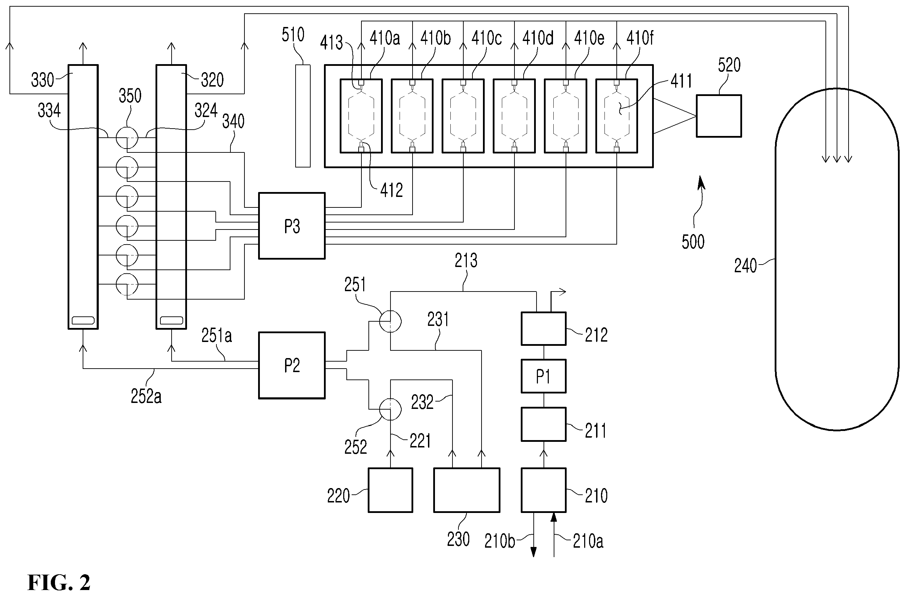

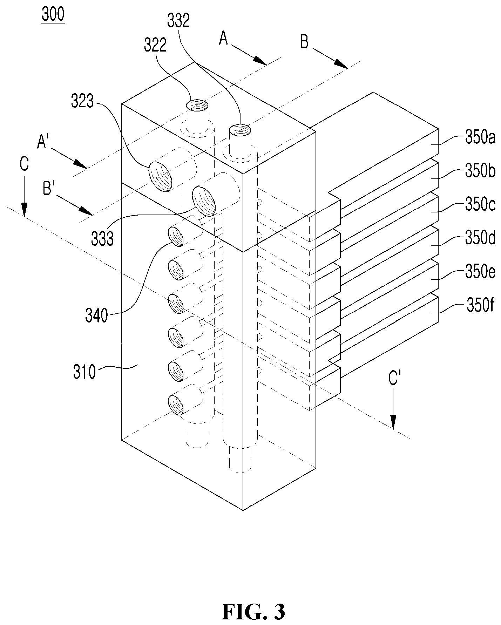

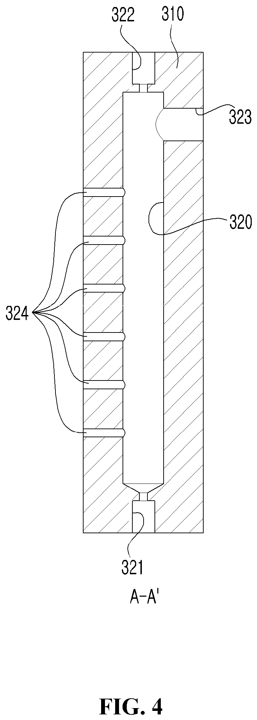

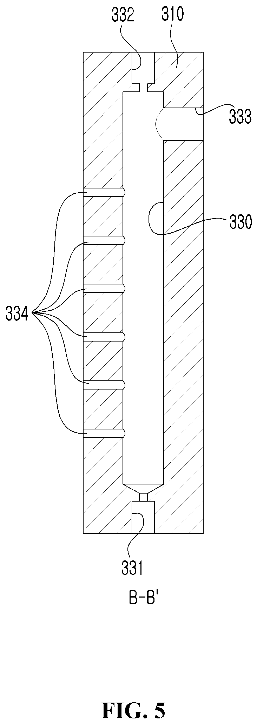

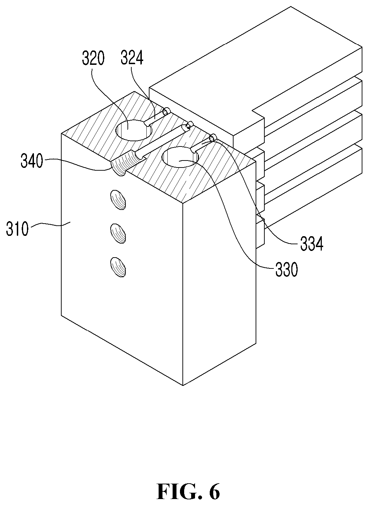

FIG. 1 is a perspective view of an online continuous biotoxicity monitoring device according to an embodiment of the present invention, FIG. 2 is a diagram of the online continuous biotoxicity monitoring device according to the embodiment of the present invention, FIG. 3 is a perspective view of a continuous dilution unit according to the embodiment of the present invention, FIG. 4 is a cross-sectional view along direction A-A' of FIG. 3, FIG. 5 is a cross-sectional view along direction B-B' of FIG. 3, and FIG. 6 is a cross-sectional perspective view along direction C-C' of FIG. 3.

Hereinafter, embodiments of the present invention will be described in detail with reference to FIGS. 1 to 6.

An online continuous biotoxicity monitoring device 1000 may include a housing 100, a supply unit 200, a continuous dilution unit 300, a chamber part 400, an optical imaging unit 500, an analysis unit, and a controller.

The housing 100 forms an external appearance of the automated toxicity test device 1000 using an aquatic organism and protects components provided inside the housing 100. When a toxicity test using an aquatic organism is performed, the housing 100 blocks influence of an external environment (a temperature, a light, or the like) and maintains the same experimental condition for each experiment.

The supply unit 200, which is provided inside the housing 100 to supply samples, culture media, and algae to the continuous dilution unit 300, includes a sample storing unit 210, a culture medium storing unit 220, a unit for storing algae 230, a waste liquid storing unit 240, and a supply valve 250.

The sample to be evaluated for toxicity is introduced into and contained in the sample storing unit 210. This sample may be usually collected from rivers on the ground surface and lakes, reservoirs, and detention pond having stagnant water or introduced in a treatment operation of a sewage treating plant, a water purifying plant, a receiving well, and a wastewater treating plant or from an effluent.

Preferably, a sample inlet pipe 210a and a sample outlet pipe 201b may be connected to the sample storing unit 210. In detail, the sample may be continuously introduced into the sample storing unit 210 along the sample inlet pipe 210a and continuously discharged to an area, in which the sample is collected, along the sample outlet pipe 210b. In addition, the sample may be continuously discharged from the sample storing unit 210 to the waste liquid storing unit 240. Accordingly, real-time changes in harmful toxic substances contained in the sample may be measured.

Preferably, a filter 211 that may filter out floating matters contained in the sample may be disposed inside the sample storing unit 210. For example, the filter 211 may include a metal filter, a ceramic filter, a fiber filter, a filtration film, and the like.

The sample introduced into the sample storing unit 210 is transferred to a sample temperature maintaining unit 212 by a first pump P1.

The sample temperature maintaining unit 212 is provided with, for example, a temperature maintaining device such as a Peltier element. Since aquatic organisms live in different environments, an optimum temperature should be maintained. The sample temperature maintaining unit 212 heats or cools the sample so that a temperature of the sample is kept constant.

In addition, the sample temperature maintaining unit 212 is provided with a bubble removing device such as an ultrasonic device. The sample temperature maintaining unit 212 removes bubbles from the sample and minimizes influence on the bubbles when photographing is performed by the optical imaging unit 500.

A culture medium for diluting the sample is stored in the culture medium storing unit 220. The culture medium is synthetic water prepared to normally cultivate toxicity test organisms.

The unit for storing algae 230 stores cultured algae, which are supplied as food for toxicity test organisms, and is provided with a refrigeration module. Accordingly, the unit for storing algae 230 may maintain an internal temperature at approximately 5 degrees that is an appropriate temperature at which the algae is not spoiled.

The waste liquid storing unit 240 stores a mixture of the sample and the algae or a mixture of the culture medium and the algae, which flows out from the continuous dilution unit 300 and the chamber part 400.

The supply valve 250 supplies the culture medium and the sample to the continuous dilution unit 300 by an operation of a second pump P2. For example, the supply valve 250 may be a three-way solenoid valve.

In detail, the sample temperature maintaining unit 212 of the sample storing unit 210 and the unit for storing algae 230 are connected to an inlet side of a first supply valve 251 by a sample storing unit line 213 and a unit for storing algae line 231, and a sample storing tank 320 is connected to an outlet side of the first supply valve 251 by a sample supply line 251a.

In more detail, the first supply valve 251 supplies the sample or the algae to the sample storing tank 320 of the continuous dilution unit 300 according to an applied current. For example, when a current is applied to the first supply valve 251, the unit for storing algae line 231 is opened, the sample storing unit line 213 is closed, and thus the algae accommodated in the unit for storing algae 230 is supplied to the sample storing tank 320. In addition, when a current is not applied to the first supply valve 251, the unit for storing algae line 231 is switched to a closed state, the sample storing unit line 213 is switched to an open state, and thus the sample accommodated in the sample temperature maintaining unit 212 is supplied to the sample storing tank 320.

Here, when an opening and closing time of the first supply valve 251 is adjusted through the controller, a concentration of the algae supplied to the sample storing tank 320 may be adjusted.

Meanwhile, the culture medium storing unit 220

and the unit for storing algae 230 are connected to an inlet side of a second supply valve 252 by a culture medium storing unit line 221 and a unit for storing algae line 232, and a culture medium storing tank 330 is connected to an outlet side of the second supply valve 252 by a culture medium supply line 252a.

In detail, the second supply valve 252 supplies the culture medium or the algae to the culture medium storing tank 330 of the continuous dilution unit 300 according to an applied current. For example, when a current is applied to the second supply valve 252, the unit for storing algae line 232 is opened, the culture medium storing unit line 221 is closed, and thus the algae accommodated in the unit for storing algae 230 is supplied to the culture medium storing tank 330. In addition, when a current is not applied to the second supply valve 252, the unit for storing algae line 232 is switched to a closed state, the culture medium storing unit line 221 is switched to an open state, and thus the culture medium accommodated in the culture medium storing unit 220 is supplied to the culture medium storing tank 330.

Here, when an opening and closing time of the second supply valve 252 is adjusted through the controller, a concentration of the algae supplied to the culture medium storing tank 330 may be adjusted.

The continuous dilution unit 300 mixes the sample and the culture medium supplied from the sample storing unit 210 and the culture medium storing unit 220 at various mixing ratios and supplies mixtures having different dilution ratios to the chamber part 400. As illustrated in FIGS. 3 to 6, the continuous dilution unit 300 includes a body 310, the sample storing tank 320, the culture medium storing tank 330, a dilution line 340, and a dilution valve 350.

The body 310 is formed to extend in a vertical direction, and the sample storing tank 320 is formed to pass through the body 31 in the vertical direction. An inlet 321 through which the sample or the algae supplied from the sample storing unit 210 and the unit for storing algae 230 is supplied is provided at a lower end of the sample storing tank 320, and a vent part 322 is formed at an upper end of the sample storing tank 320. In addition, an outlet 323 which communicates with the sample storing tank 320 and through which an oversupplied mixture of the sample and the algae is discharged to the waste liquid storing unit 240 is formed at a front side of an upper end of the body 310.

A plurality of sample dispensing lines 324, which are formed to pass in a horizontal direction, communicate with the sample storing tank 320, and are spaced apart from each other from an upper side to a lower side of the body 310, are formed on a rear side of the body 310. Each of the sample dispensing lines 324 is fluidly connected to an inlet side of the dilution valve 350.

In this way, as the plurality of sample dispensing lines 324 are formed in one body 310, the sample may be supplied to a plurality of dilution valves 350 at a constant flow rate.

The culture medium storing tank 330 is formed to pass along the body 310 of the continuous dilution unit 300 in the vertical direction and is formed to be horizontal to the sample storing tank 320. An inlet 331, through which the culture medium or the algae supplied from the culture medium storing unit 220 and the unit for storing algae 230 are supplied, is provided at a lower end of the culture medium storing tank 330, and a vent part 332 is formed at an upper end of the culture medium storing tank 330. In addition, an outlet 333, which communicates with the culture medium storing tank 330 and through which an oversupplied mixture of the culture medium and the algae is discharged to the waste liquid storing unit 240, is formed at the front side of the upper end of the body 310.

A plurality of culture medium dispensing lines 334, which are formed to pass in the horizontal direction, communicate with the culture medium storing tank 330, and are spaced apart from each other from the upper side to the lower side of the body 310, are formed on the rear side of the body 310. Each of the culture medium dispensing lines 334 is fluidly connected to the inlet side of the dilution valve 350.

In this way, as the plurality of culture medium dispensing lines 334 are formed in one body 310, the sample may be supplied to the plurality of dilution valves 350 at a constant flow rate.

The dilution line 340 is formed between the sample dispensing line 324 and the culture medium dispensing line 334 to pass from a rear side to a front side in the horizontal direction of the body 310 of the continuous dilution unit 300. In addition, the plurality of dilution lines 340 are formed to be spaced apart from each other from the upper side to the lower side and are fluidly connected to an outlet side of the dilution valve 350.

The dilution valve 350 dilutes the culture medium and the sample at a certain ratio by an operation of a third pump P3, and may be, for example, a three-way solenoid valve. The sample dispensing line 324 and the culture medium dispensing line 334 are fluidly connected to the inlet side of the dilution valve 350, and the dilution line 340 is fluidly connected to the outlet side of the dilution valve 350.

For example, when no current is applied to the dilution valve 350, the sample dispensing line 324 is maintained in an open state, the culture medium dispensing line 334 is maintained in a closed state, and thus the sample accommodated in the sample storing tank 320 moves to the dilution line 340 along the sample dispensing line 324. For example, when a current is applied to the dilution valve 350, the sample dispensing line 324 is switched to a closed state, the culture medium dispensing line 334 is switched to an open state, and thus the culture medium accommodated in the culture medium storing tank 330 flows to the dilution line 340 along the culture medium dispensing line 334.

That is, when an opening and closing time of the dilution valve 350 is adjusted, a concentration of the sample may be adjusted.

Preferably, the dilution valve 350 may include a first dilution valve 350a to a sixth dilution valve 350f that dilute the sample to 100% (undiluted solution), 50%, 25%, 12.5%, 6.25%, and 0% (100% of the culture medium).

Here, it is assumed that the concentration of the sample supplied through the sample dispensing line 324 is 100%, the concentration of the culture medium supplied through the culture medium dispensing line 334 is 100%, and the sample and the culture medium are supplied at the same flow rate.

For example, the first dilution valve 350a is controlled so that the sample dispensing line 324 is continuously maintained in an open state, and thus the sample having a concentration of 100% is supplied to the first chamber 350a.

The second dilution valve 350b is controlled so that the sample dispensing line 324 and the culture medium dispensing line 334 are maintained in an open state for the same amount of time, and thus the sample having a concentration of 50% is supplied to the second chamber 350b.

The third dilution valve 350c is controlled so that the sample dispensing line 324 and the culture medium dispensing line 334 are maintained in an open state at a ratio of 1:3, and thus the sample having a concentration of 25% is supplied to the third chamber 350c.

The fourth dilution valve 350d is controlled so that the sample dispensing line 324 and the culture medium dispensing line 334 are maintained in an open state at a ratio of 1:7, and thus the sample having a concentration of 12.5% is supplied to the fourth chamber 350d.

The fifth dilution valve 350e is controlled so that the sample dispensing line 324 and the culture medium dispensing line 334 are maintained in an open state at a ratio of 1:15, and thus the sample having a concentration of 6.25% is supplied to the fifth chamber 350e.

The sixth dilution valve 350f is controlled so that the culture medium dispensing line 334 is continuously maintained in an open state, and thus the culture medium having a concentration of 100%, that is, the sample having a concentration of 0%, is supplied to the sixth chamber 350f.

However, it is obvious that the number of dilution valves 350 is not limited to six, and various numbers of dilution valves 350 may be applied.

The chamber part 400 includes a plurality of chamber 410 and has a predetermined accommodation space 411 in which aquatic organisms may swim. It is preferable that the aquatic organisms include water fleas, but the present invention is not limited thereto, and various organisms such as zooplanktons, fishes, and algae may be applied.

An inlet 412 that fluidly communicates with the dilution line 340 is provided at a lower end of the chamber 410, and the mixed water of the sample and the culture medium supplied from the continuous dilution unit 300 is introduced through the inlet 412.

An outlet 413 that fluidly communicates with the waste liquid storing unit 240 is provided at an upper end of the chamber 410, and the mixed water of the sample and the culture medium is discharged to the waste liquid storing unit 240 through the outlet 413.

The chamber part 400 may include a first chamber 410a to a sixth chamber 410f as illustrated, but the present invention is not limited thereto, and it is obvious that the various numbers of chambers corresponding to the number of dilution valves 350 may be applied.

Preferably, the chamber 410 may have a hexagonal shape. When the accommodation space 411 inside the chamber 410 is formed in a hexagonal shape, air bubbles that may occur when the mixed water of the sample and the culture medium is introduced through the inlet 412 may be guided to the upper side of the chamber 410 along a lower inner wall of the chamber 410. In addition, the air bubbles may be guided to the outlet 413 positioned at an upper end of the chamber 410 along an upper inner wall of the chamber 410.

That is, when a cross section of the chamber part 400 is formed in a hexagonal shape, the air bubbles that interfere with photographing by the optical imaging unit 500 may be guided to the outlet 413 of the chamber part 400 and removed, and thus an influence of the air bubbles may be minimized.

Preferably, a rear surface of the chamber part 400 may be made of a translucent material, and a front surface of the chamber part 400 may be made of a transparent material.

The optical imaging unit 500 observes movement of the aquatic organisms inside the chamber part 400 and includes a lighting device 510 and an optical photographing device 520.

The lighting device 510 illuminates an inside of the chamber part 400 with a predetermined quantity of light. In this case, the lighting device 510 is provided on a rear side of the chamber part 400, and the light radiated from the lighting device 510 uniformly illuminates the accommodation space 411 inside the chamber part 400 through the rear surface of the chamber part 400, which is made of a translucent material.

The optical photographing device 520 may be as an optical camera apparatus that may project an image and store the image in a digital manner, such as a charge coupled device (CCD) or a complementary metal-oxide-semiconductor (CMOS) and is provided on a front side of the chamber part 400 to photograph the accommodation space 411 inside the transparent chamber 410.

Preferably, a plurality of optical camera apparatuses may be provided to take videos of different chambers 410, but the present invention is not limited thereto, and the entire chamber part 400 may be photographed with one optical camera apparatus.

The image captured by the optical imaging unit 500 is transmitted to the analysis unit. In this case, it is obvious that the image may be transmitted through a wired connection line or a wireless manner.

The analysis unit analyzes a toxic index (TI) for responses of organisms induced by toxic substances based on the image transmitted from the optical imaging unit 500.

In detail, the analysis unit traces swimming trajectories of the aquatic organisms from images including a plurality of frames and derives a TI value such as speed, acceleration, speed distribution, position distribution in the chamber, activity radius, total movement distance, intermittent hopping frequency, submersion rate, movement time, stopping time, movement trajectory, rotational angle, and repetitive behavior pattern of the aquatic organisms. The TI value obtained by the analysis unit is transmitted to a prediction unit.

Preferably, the analysis unit may generate an integrated TI value obtained by integrating one or more of the plurality of TI values and transmit the generated integrated TI value to the prediction unit. For example, the integrated TI value may be generated by changing a weight for each parameter. In this case, it is obvious that the TI value may be transmitted through a wired connection line or a wireless manner.

The controller opens and closes not only the first supply valve 251 and the second supply valve 252 of the supply unit 20 to adjust the concentration of algae, and but also the first dilution valve 350a to the sixth dilution valve 350f of the continuous dilution unit 300 to control a dilution ratio of the sample.

Meanwhile, TU is calculated by measuring EC50, which is determined by exposing water flea larvae for 24 hours to an effluent solution (100%) and solutions diluted to 50%, 25%, 12.5%, 6.25%, and 0%, and estimating the concentration expected to have an adverse effect on 50% of test organisms, such as lethal effect or swimming inhibition.

$$\text{Toxic Unit (TU)} = 100 \,/\, \text{EC50 (\%)}$$

For example, the ecotoxicity value of 1 TU for the sample means that 50% of the aquatic organisms exposed to the sample are killed or immobilized after 24 hours.

However, because it takes 24 hours to measure TU, manual ecotoxicity testing has the limitation of not being able to respond quickly to the inflow of toxic substances.

Thus, the online continuous biotoxicity monitoring device 1000 according to the embodiment of the present invention includes the prediction unit that predicts TU after 24 hours using the TI value.

Specifically, the prediction unit outputs the predicted TU at a future target time, that is, 24 hours later, from the TI value measured at the initial point of introduction of the toxic substances through the TU prediction model. The predicted TU refers to the TU expected by the TI value measured over certain period of time.

Meanwhile, a correlation between TI data and time is stored in advance in the TU prediction model. Here, the TI data includes some or all of the TI values such as speed, acceleration, speed distribution, position distribution within the chamber, activity radius, total movement distance, intermittent hopping frequency, submersion rate, movement time, stopping time, movement trajectory, rotational angle, and repetitive behavior pattern of the aquatic organisms. Preferably, the correlation between time and integrated TI data obtained by integrating one or more of TI values may be stored in advance in the prediction unit.

Preferably, when the integrated TI value is input, the prediction unit compares the input integrated TI value with the previously stored integrated TI data and predicts whether the aquatic organisms are killed or immobilized after 24 hours.

In addition, the prediction unit identifies the concentrations predicted to affect a half of the aquatic organisms among the sample solution 100% and the samples diluted to 50%, 25%, 12.5%, 6.25%, and 0% and calculates the predicted TU through Equation {TU = 100/EC50}.

In this way, the online continuous biotoxicity monitoring device 1000 according to the embodiment of the present invention can be miniaturized by supplying samples of different concentrations to the chamber unit 400 through the simple continuous dilution unit 300.

In addition, the TI of the sample flowing into the chamber part 400 is measured in real time, and the predicted TU at the future target time, that is, after 24 hours, can be calculated from the TI value measured at the beginning of 24 hours. If hazardous substances are introduced and the TU exceeds the effluent standard, the operators can respond quickly to the hazardous substances.

The above description of the present invention is merely illustrative, and those skilled in the art to which the present invention pertains can understand that the present invention can be easily modified in other specific forms . Therefore, it should be understood that the embodiments described above are illustrative but not limiting in all aspects. For example, components described as a single type may be implemented in a distributed manner, and likewise, components described as a distributed manner may also be implemented in a coupled form.

Description of reference numerals

1000: Online continuous biotoxicity monitoring device
100: Housing
200: Supply unit
300: Continuous dilution unit

400: Chamber part
500: Optical imaging unit

**Claims**

1. An online continuous biotoxicity monitoring device (1000) comprising:

   a housing (100);
   a supply unit (200) including a sample storing unit (210) in which a sample is stored and a culture medium storing unit (220) in which a culture medium configured to dilute the sample is stored;
   a continuous dilution unit (300) configured to mix the sample and the culture medium supplied from the sample storing unit (210) and the culture medium storing unit (220) at various mixing ratios, including a sample storing tank (320) in which the sample supplied from the sample storing (210) unit is stored, a culture medium storing tank (330) in which the culture medium supplied from the culture medium storing unit (220) is stored, and a plurality of dilution valves (350) connected to a plurality of sample dispensing lines (324) dispensed from the sample storing tank (320) and a plurality of culture medium dispensing lines (334) dispensed from the culture medium storing tank (330) and controlled to supply the sample or the culture medium at different dilution ratios;
   a chamber part (400) in which an aquatic organism is accommodated and which continuously receives a mixture of the sample and the culture medium through the continuous dilution unit (300);
   an optical imaging unit (500) configured to take a video of activity of the aquatic organism; and
   an analysis unit configured to evaluate toxicity of the sample through the captured image transmitted from the optical imaging unit (500).

2. The online continuous biotoxicity monitoring device of claim 1, wherein the continuous dilution unit (300) further includes:

   a body (310) extending in a vertical direction; and
   a plurality of dilution lines (340) passing from a rear wide to a front side of the body and connected to the chamber part (400) and the dilution valves (350),
   the sample storing tank (320) and the culture medium storing tank (330) are formed to pass through the body (310) in the vertical direction,
   the sample dispensing lines (324) pass from the sample storing tank (320) to a rear side of the body (310) and are spaced apart from each other in the vertical direction, and
the culture medium dispensing lines (334) pass from the culture medium storing tank (330) to the rear side of the body (310) and are spaced apart from each other in the vertical direction.

3. The online continuous biotoxicity monitoring device of claim 1 or 2, wherein the dilution valve (350) includes a first dilution valve to a sixth dilution valve.

4. The online continuous biotoxicity monitoring device of one of claims 1 to 3, wherein the supply unit (200) further includes:
   a unit for storing algae (230) where algae supplied as food for toxicity test organisms are stored.

5. The online continuous biotoxicity monitoring device of claim 4, wherein the supply unit (200) further includes:

   a first supply valve (251) of which an inlet is connected to the sample storing unit (210) and the unit for storing algae (230) and an outlet is connected to the sample storing tank (320) and which is controlled to supply the sample or the algae to the sample storing tank (320); and
   a second supply valve (252) of which an inlet is connected to the culture medium storing unit (220) and the unit for storing algae (230) and an outlet is connected to the culture medium storing tank (330) and which is controlled to supply the culture medium or the algae to the culture medium storing tank (330).

6. The online continuous biotoxicity monitoring device of one of claims 1 to 4, wherein an interior of the chamber part (400) has a hexagonal shape.

7. The online continuous biotoxicity monitoring device of claim 6, wherein an inlet through which the mixture of the sample and the culture medium is introduced is provided at a lower end of the chamber part (400) and an outlet through which the mixture of the sample and the culture medium is discharged is provided at an upper end of the chamber part (400).

8. The online continuous biotoxicity monitoring device of one of claims 1 to 7, wherein a front side of the chamber part (400) is made of a transparent material, and
   a rear side of the chamber part (400) is made of a translucent material.

9. The online continuous biotoxicity monitoring device of claim 8, wherein the optical imaging unit (500) includes:

a lighting device (510) disposed behind the chamber part (400); and

an optical camera device (520) provided in front of the chamber part (400) and configured to photograph an accommodation space (410) inside the chamber part (400).

10. The online continuous biotoxicity monitoring device of one of claims 1 to 9, wherein the analysis unit is configured to measure a toxic index (TI) of the sample.

11. The online continuous biotoxicity monitoring device of claim 10, further comprising:

a prediction unit configured to calculate a predicted toxic unit (TU) based on the TI measurement value transmitted from the analysis unit.

12. The online continuous biotoxicity monitoring device of claim 11, wherein the prediction unit is configured to calculate the predicted TU by comparing the TI measurement value with previously stored TI data.

**Patentansprüche**

1. Vorrichtung (1000) zur kontinuierlichen Online-Überwachung der Biotoxizität, umfassend:

ein Gehäuse (100);

eine Zuführungseinheit (200), die eine Probenspeichereinheit (210) aufweist, in der eine Probe gespeichert ist, und eine Kulturmediumspeichereinheit (220), in der ein Kulturmedium, das dazu konfiguriert ist, die Probe zu verdünnen, gespeichert ist;

eine kontinuierliche Verdünnungseinheit (300), die dazu konfiguriert ist, die Probe und das von der Probenspeichereinheit (210) und der Kulturmediumspeichereinheit (220) gelieferte Kulturmedium in verschiedenen Mischverhältnissen zu mischen, und die einen Probenspeichertank (320) aufweist, in dem die von der Probenspeichereinheit (210) gelieferte Probe aufbewahrt wird, einen Kulturmediumspeichertank (330), in dem das von der Kulturmediumspeichereinheit (220) gelieferte Kulturmedium gespeichert wird, und eine Vielzahl von Verdünnungsventilen (350), die mit einer Vielzahl von Probenabgabeleitungen (324), die von dem Probenspeichertank (320) abgehen, und einer Vielzahl von Kulturmediumabgabeleitungen (334), die von dem Kulturmediumspeichertank (330) abgehen, verbunden sind, und die gesteuert werden, um die Probe oder das Kulturmedium in unterschiedlichen Verdünnungsverhältnissen zuzuführen;

einen Kammerteil (400), in dem ein Wasserorganismus untergebracht ist und der kontinuierlich eine Mischung aus der Probe und dem Kulturmedium durch die kontinuierliche Verdünnungseinheit (300) erhält;

eine optische Abbildungseinheit (500), die dazu konfiguriert ist, ein Video der Aktivität des Wasserorganismus aufzunehmen; und

eine Analyseeinheit, die dazu konfiguriert ist, die Toxizität der Probe anhand des aufgenommenen Bildes, das von der optischen Abbildungseinheit (500) übertragen wird, zu evaluieren.

2. Vorrichtung zur kontinuierlichen Online-Überwachung der Biotoxizität nach Anspruch 1, wobei die kontinuierliche Verdünnungseinheit (300) des Weiteren aufweist:

einen Körper (310), der sich in vertikaler Richtung erstreckt; und

eine Vielzahl von Verdünnungsleitungen (340), die von einer Rückseite zu einer Vorderseite des Körpers verlaufen und mit dem Kammerteil (400) und den Verdünnungsventilen (350) verbunden sind,

wobei der Probenspeichertank (320) und der Kulturmediumspeichertank (330) so ausgebildet sind, dass sie durch den Körper (310) in der vertikalen Richtung verlaufen,

die Probenabgabeleitungen (324) von dem Probenspeichertank (320) zu einer Rückseite des Körpers (310) verlaufen und in der vertikalen Richtung voneinander beabstandet sind, und

die Kulturmediumabgabeleitungen (334) von dem Kulturmediumspeichertank (330) zu der Rückseite des Körpers (310) verlaufen und in der vertikalen Richtung voneinander beabstandet sind.

3. Vorrichtung zur kontinuierlichen Online-Überwachung der Biotoxizität nach Anspruch 1 oder 2, wobei das Verdünnungsventil (350) ein erstes Verdünnungsventil bis ein sechstes Verdünnungsventil aufweist.

4. Vorrichtung zur kontinuierlichen Online-Überwachung der Biotoxizität nach einem der Ansprüche 1 bis 3, wobei die Zuführungseinheit (200) des Weiteren Folgendes aufweist:

eine Einheit zum Speichern von Algen (230), in der Algen gespeichert sind, die als Nahrung für Toxizitätstestorganismen dienen.

5. Vorrichtung zur kontinuierlichen Online-Überwachung der Biotoxizität nach Anspruch 4, wobei die Zuführungseinheit (200) des Weiteren aufweist:

ein erstes Zuführungsventil (251), von dem ein Einlass mit der Probenspeichereinheit (210) und der Einheit zum Speichern von Algen (230) verbunden ist, und ein Auslass mit dem Probenspeichertank (320) verbunden ist und das gesteuert wird, um die Probe oder die Algen dem Probenspeichertank (320) zuzuführen; und ein zweites Zuführungsventil (252), von dem ein Einlass mit der Kulturmediumspeichereinheit (220) und der Einheit zum Speichern von Algen (230) verbunden ist und ein Auslass mit dem Kulturmediumspeichertank (330) verbunden ist und das gesteuert wird, um das Kulturmedium oder die Algen dem Kulturmediumspeichertank (330) zuzuführen.

6. Vorrichtung zur kontinuierlichen Online-Überwachung der Biotoxizität nach einem der Ansprüche 1 bis 4, wobei ein Innenraum des Kammerteils (400) eine hexagonale Form aufweist.

7. Vorrichtung zur kontinuierlichen Online-Überwachung der Biotoxizität nach Anspruch 6, wobei ein Einlass, durch den das Gemisch aus der Probe und dem Kulturmedium eingeführt wird, an einem unteren Ende des Kammerteils (400) vorgesehen ist, und ein Auslass, durch den das Gemisch aus der Probe und dem Kulturmedium abgeführt wird, an einem oberen Ende des Kammerteils (400) vorgesehen ist.

8. Vorrichtung zur kontinuierlichen Online-Überwachung der Biotoxizität nach einem der Ansprüche 1 bis 7, wobei eine Vorderseite des Kammerteils (400) aus einem transparenten Material hergestellt ist, und eine Rückseite des Kammerteils (400) aus einem transluzenten Material besteht.

9. Vorrichtung zur kontinuierlichen Online-Überwachung der Biotoxizität nach Anspruch 8, wobei die optische Abbildungseinheit (500) aufweist:

   eine Beleuchtungsvorrichtung (510), die hinter dem Kammerteil (400) angeordnet ist; und eine optische Kameravorrichtung (520), die vor dem Kammerteil (400) vorgesehen und dazu konfiguriert ist, einen Aufnahmeraum (410) innerhalb des Kammerteils (400) zu fotografieren.

10. Vorrichtung zur kontinuierlichen Online-Überwachung der Biotoxizität nach einem der Ansprüche 1 bis 9, wobei die Analyseeinheit dazu konfiguriert ist, einen Toxizitätsindex (TI) der Probe zu messen.

11. Vorrichtung zur kontinuierlichen Online-Überwachung der Biotoxizität nach Anspruch 10, des Weiteren umfassend:
   eine Vorhersageeinheit, die dazu konfiguriert ist, basierend auf dem von der Analyseeinheit übertragenen TI-Messwert eine vorhergesagte toxische Einheit (TU) zu berechnen.

12. Vorrichtung zur kontinuierlichen Online-Überwachung der Biotoxizität nach Anspruch 11, wobei die Vorhersageeinheit dazu konfiguriert ist, die vorhergesagte TU durch Vergleichen des TI-Messwertes mit zuvor gespeicherten TI-Daten zu berechnen.

## Revendications

1. Dispositif de surveillance continue de la biotoxicité en ligne (1000) comprenant:

   un logement (100);
   une unité d'alimentation (200) comprenant une unité de stockage d'échantillon (210) dans laquelle est stocké un échantillon et une unité de stockage de milieu de culture (220) dans laquelle est stocké un milieu de culture configuré pour diluer l'échantillon;
   une unité de dilution continue (300) configurée pour mélanger l'échantillon et le milieu de culture fournis par l'unité de stockage d'échantillon (210) et l'unité de stockage de milieu de culture (220) selon différents rapports de mélange, comprenant un réservoir de stockage d'échantillon (320) dans lequel est stocké l'échantillon fourni par l'unité de stockage d'échantillon (210), un réservoir de stockage de milieu de culture (330) dans lequel est stocké le milieu de culture fourni par l'unité de stockage de milieu de culture (220), et une pluralité de vannes de dilution (350) reliées à une pluralité de lignes de distribution d'échantillon (324) distribuées à partir du réservoir de stockage d'échantillon (320) et une pluralité de lignes de distribution de milieu de culture (334) distribuées à partir du réservoir de stockage de milieu de culture (330) et commandées pour fournir l'échantillon ou le milieu de culture selon différents rapports de dilution;
   une partie chambre (400) dans laquelle est logé un organisme aquatique et qui reçoit en continu un mélange de l'échantillon et du milieu de culture par l'intermédiaire de l'unité de dilution continue (300);
   une unité d'imagerie optique (500) configurée enregistrer une vidéo de l'activité de l'organisme aquatique; et
   une unité d'analyse configurée pour évaluer la toxicité de l'échantillon à partir de l'image capturée et transmise par l'unité d'imagerie optique (500).

2. Dispositif de surveillance continue de la biotoxicité en ligne selon la revendication 1, dans lequel l'unité de dilution continue (300) comprend en outre:

un corps (310) s'étendant dans une direction verticale; et

une pluralité de lignes de dilution (340) passant d'une partie arrière large à une partie avant du corps et reliées à la partie chambre (400) et aux vannes de dilution (350),

le réservoir de stockage d'échantillon (320) et le réservoir de stockage de milieu de culture (330) sont formés de manière à traverser le corps (310) dans la direction verticale,

les lignes de distribution d'échantillon (324) passent du réservoir de stockage d'échantillon (320) vers un côté arrière du corps (310) et sont espacées les unes des autres dans la direction verticale, et

les lignes de distribution de milieu de culture (334) passent du réservoir de stockage de milieu de culture (330) vers un côté arrière du corps (310) et sont espacées les unes des autres dans la direction verticale.

3. Dispositif de surveillance continue de la biotoxicité en ligne selon la revendication 1 ou 2, dans lequel la vanne de dilution (350) comprend une première valve de dilution jusqu'à une sixième valve de dilution.

4. Dispositif de surveillance continue de la biotoxicité en ligne selon l'une des revendications 1 à 3, dans lequel l'unité d'alimentation (200) comprend en outre:
une unité de stockage d'algues (230) où sont stockées les algues fournies comme nourriture pour les organismes de test de toxicité.

5. Dispositif de surveillance continue de la biotoxicité en ligne selon la revendication 4, dans lequel l'unité d'alimentation (200) comprend en outre:

une première vanne d'alimentation (251) dont une entrée est reliée à l'unité de stockage d'échantillon (210) et à l'unité de stockage d'algues (230), et une sortie est reliée au réservoir de stockage d'échantillon (320) et qui est commandée pour alimenter le réservoir de stockage d'échantillons (320) en échantillon ou en algues; et
une deuxième vanne d'alimentation (252), dont une entrée est reliée à l'unité de stockage de milieu de culture (220) et à l'unité de stockage d'algues (230) et une sortie est reliée au réservoir de stockage de milieu de culture (330) et qui est commandée pour alimenter le réservoir de stockage de milieu de culture (330) en milieu de culture ou en algues.

6. Dispositif de surveillance continue de la biotoxicité en ligne selon l'une des revendications 1 à 4, dans lequel l'intérieur de la partie chambre (400) a une forme hexagonale.

7. Dispositif de surveillance continue de la biotoxicité en ligne selon la revendication 6, dans lequel une entrée par laquelle le mélange de l'échantillon et du milieu de culture est introduit est prévue à l'extrémité inférieure de la partie chambre (400) et une sortie par laquelle le mélange de l'échantillon et du milieu de culture est évacué est prévue à une extrémité supérieure de la partie chambre (400).

8. Dispositif de surveillance continue de la biotoxicité en ligne selon l'une des revendications 1 à 7, dans lequel une face avant de la partie chambre (400) est constituée d'un matériau transparent, et
une face arrière de la partie chambre (400) est constituée d'un matériau translucide.

9. Dispositif de surveillance continue de la biotoxicité en ligne selon la revendication 8, dans lequel l'unité d'imagerie optique (500) comprend:

un dispositif d'éclairage (510) disposé derrière la partie chambre (400); et
un dispositif de caméra optique (520) prévu à l'avant de la partie chambre (400) et configuré pour photographier un espace d'hébergement (410) à l'intérieur de la partie chambre (400).

10. Dispositif de surveillance continue de la biotoxicité en ligne selon l'une des revendications 1 à 9, dans lequel l'unité d'analyse est configurée pour mesurer un indice de toxicité (IT) de l'échantillon.

11. Dispositif de surveillance continue de la biotoxicité en ligne selon la revendication 10, comprenant en outre:
une unité de prédiction configurée pour calculer une unité toxique (UT) prédite basée sur la valeur de mesure de l'IT transmise par l'unité d'analyse.

12. Dispositif de surveillance continue de la biotoxicité en ligne selon la revendication 11, dans lequel l'unité de prédiction est configurée pour calculer l'UT prédite en comparant la valeur de mesure de l'IT avec les données de l'IT précédemment stockées.

**FIG. 1**

FIG. 2

**FIG. 3**

FIG. 4

FIG. 5

**FIG. 6**

**EP 4 386 379 B1**

**Patent documents cited in the description**

- KR 102317085 B1
- KR 101366786 B1